(19) Europäisches Patentamt European Patent Office Office européen des brevets

(11) **EP 3 400 978 B1**

(12) **EUROPEAN PATENT SPECIFICATION**

(45) Date of publication and mention
of the grant of the patent:
**19.11.2025 Bulletin 2025/47**

(21) Application number: **17170297.0**

(22) Date of filing: **09.05.2017**

(51) International Patent Classification (IPC):
**A61M 1/28** (2006.01)

(52) Cooperative Patent Classification (CPC):
**A61M 1/28;** A61M 2205/3393; A61M 2205/3546;
A61M 2205/3553; A61M 2205/368; A61M 2209/082;
A61M 2209/084

(54) **CONTINUOUS AMBULATORY DIALYSIS SYSTEM**

SYSTEM ZUR KONTINUIERLICHEN AMBULANTEN DIALYSE

SYSTÈME DE DIALYSE CONTINUE AMBULATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.11.2018 Bulletin 2018/46**

(73) Proprietor: **Polifarma Ilaç Sanayi ve Tic A.S.
Istanbul (TR)**

(72) Inventor: **IVEGEN, Osman Murat
Tekirdag (TR)**

(74) Representative: **Mutlu, Aydin
Invokat Intellectual Property Services Ltd.
Kartaltepe Mh. Yildiztepe Sk.
No:6-Bakirköy
34145 Istanbul (TR)**

(56) References cited:
**EP-A1- 2 623 139      US-A- 5 445 610
US-A1- 2016 216 150**

## Description

## Field of the Invention

The present invention relates to a gravimetric type continuous ambulatory peritoneal dialysis device. The invention particularly relates to a continuous ambulatory peritoneal dialysis device and a system thereof which is provided with automatization by means of controlled valves, and storing the dialysate data in a remote database, allowing a physician to monitor the treatment process and observe commitment of the patient.

## Background of the Invention

Kidney failure and reduced kidney function have been treated with dialysis. Dialysis removes waste, toxins and excess water from the body which is normally the functioning of the kidneys. Dialysis treatment for the replacement of kidney's function is essential for many of the patients because the treatment is crucial. Hemodialysis and peritoneal dialysis are the two types of dialysis therapies commonly used to treat toxins caused by loss of kidney function.

Peritoneal dialysis (PD) utilizes a dialysis solution or dialysate, which is infused into a patient's peritoneal cavity. The dialysate contacts with the peritoneal membrane of the peritoneal cavity. Diffusion and osmosis exchanges take place between the solution and the bloodstream across the peritoneal membrane. Therefore, waste, toxins and excess water pass from the patient's bloodstream, through the peritoneal membrane into the dialysate. The dialysate consumed in the peritoneal cavity is drained from the abdomen of the patient, removing wastes, toxins and excess water from the blood. This cycle can be repeated during the night in which case an *automated dialysis machine* is used because the patient falls asleep. This cycle may also be repeated during the day which is generally called a *"continuous ambulatory peritoneal dialysis" (CAPD)*.

Continuous ambulatory peritoneal dialysis (CAPD) is a dialysis system that can continue for 24 hours. Patients can walk, lie down or work during dialysis. In this system, approximately 2 litres of dialysate is infused to peritoneum with peritoneal dialysis catheter. Dialysis begins when the dialysate is drained into the peritoneum. The removal process takes place in this membrane in the abdomen of the patients, the undesirable substances are purified within this period. This process is called the "waiting period-(Dwell)". After, the dirty dialysate fluid is drained from the abdomen and this draining takes about 20-30 minutes. Thereafter, new sterile fluid is reloaded to the abdomen by means of gravity for about 6-8 minutes and process begins again. This cycle continues throughout the day as the doctor decides.

Continuous ambulatory peritoneal dialysis is not as successful as healthy kidneys in drainage of the fluid. Therefore, there is a "dry weight" which should be maintained during the process. In this weight, it is necessary that the blood pressure should be at normal level and should neither be overloaded with fluid nor loaded with less fluid. Fluid overload causes working of heart to be harder and high blood pressure. When high blood pressure becomes permanent, it may cause a constant tension on the heart and increase the risk of heart attack. For this reason, it is crucial to keep control of the amount of fluid received and given by patient in order to minimize the risk of undesirable heart diseases.

Continuous ambulatory peritoneal dialysis is a manual system because it is carried out by the patient including the all steps of replacing of dialysis bag and monitoring the weights. Therefore, the CAPD process is open to errors. The patient can start the next cycle before completion or after a long time of completion of the dialysis cycles, and may have difficulties in adjusting retention of the desired amount of dialysis fluid in the peritoneal cavity within a desired period. Additionally, it is difficult for the doctor to control of whether said dialysis cycles have been done properly.

As a partial solution to the foregoing problems, a system has been disclosed in US 2016/0216150, which aims to determine phase changes and alert the patient accordingly by incorporating sensors, particularly weight sensors, to a peritoneal dialysis device. Accordingly, for example, a weight sensor is installed in connection with the dialysate bag, and a weight sensor is installed in connection with the discharge bag so as to continuously monitor the amount of fluid in the bags. The system produces a warning when the fluid in the dialysate bag is drained when the filling process is completed. Likewise, when the amount of fluid in the discharge bag reaches the desired level, a warning is generated that the drainage process is over. However, in this system, the filling and discharging cycles are left to the patient's control and thus the medical staff encounters difficulties in monitoring the dialysis cycles.

The present invention overcomes the foregoing problems by providing a continuous ambulatory peritoneal dialysis system which is safer for usage of the patient, easy to use and being also convenient for the follow up of medical staff.

## Brief Description of the Invention

A continuous ambulatory peritoneal dialysis device according to the present invention comprises the technical features as defined in independent claim 1. A continuous ambulatory peritoneal dialysis system according to the present invention comprises the technical features as defined in independent claim 9.

The present disclosure provides a continuous ambulatory peritoneal dialysis device (1), a peritoneal dialysis system which involves said device, and a method for collecting and processing the data obtained through the operation of the foregoing in order to overcome above mentioned difficulties and problems. The continuous ambulatory peritoneal dialysis device (1) according to the present invention comprises a body (6) having suitable areas for hanging a dialysis fluid bag (7a) and discharge bag (7b), a first weight sensor (4a) in the area where the dialysis fluid bag (7a) is hanged upon which measures the weight of the bag (7a) and generates a signal proportional thereto, a second weight sensor (4b) in the area where the discharge bag (7b) is hanged upon which measures the weight of the bag (7b) and generates a signal proportional thereto and a dialysis fluid valve (2a) and a dialysate valve (2b) provided on said body (6). The device also comprises a processor (2c) which generates a control signal to close the dialysis fluid valve (2a) when the signal received from first weight sensor (4a) is equal or less than a predetermined value, and to close the dialysate valve (2b) when the signal received from second weight sensor (4b) is equal or greater than a predetermined value. In addition, the device further comprises a mobile device (3) receiving signals from said weight sensors (4a, 4b) and generating a filtration data from the difference of these signals.

According to the present invention, said filtration data is filtration weight ($m_F$), wherein;

$$m_F = (m_{Df} - m_{Di}) - (m_{DSi} - m_{DSf})$$

and wherein;

$m_{DSi}$ = the weight of the initially filled dialysate fluid bag (7a),

$m_{DSf}$ = the weight of the drained dialysis fluid bag (7a),

$m_{Di}$ = the weight of the initially empty discharge bag (7a),

$m_{Df}$ = the weight of the discharge bag (7b) filled with dialysate.

Said mobile device (3) may comprise a memory for storing the filtration data based on time. This mobile device (3) can be a computer, laptop, smart phone or tablet. The communication between said mobile device (3) and the weight sensors (4a, 4b) can be a wireless communication means including Bluetooth, Wi-Fi, Infrared or an optical communication protocol. Similarly, the communication between processor (2c) and the weight sensors (4a, 4b) can be a wireless communication means including Bluetooth, Wi-Fi, Infrared or an optical communication protocol.

The continuous ambulatory peritoneal dialysis device (1) according to the present invention may further comprise a heater (9). Said heater (9) may comprise an inlet conduit (91) and an outlet conduit (92) for instantaneous and in situ heating during dialysis fluid flow. This heater (9) may comprise a heating device operated with electrical resistance, induction or radiation principles. This heater (9) preferably comprises a halogen lamp.

In another aspect, the invention provides a continuous ambulatory peritoneal dialysis system comprising a continuous ambulatory peritoneal dialysis device (1) having a mobile device (3), a server in communication with the device (1) and/or mobile device (3), a database (100) connected to said server and at least a terminal device (102) in communication with said server (3). Said database (100) can store time-dependent filtration data. Hereby, the mobile device (3), continuous ambulatory peritoneal dialysis device (1) and/or terminal device (102) can comprise an internet communication protocol for communication with the server (3).

In another aspect, the present disclosure further provides a method for data collection and processing pertaining to the continuous ambulatory peritoneal dialysis, comprising the steps of:

- providing a continuous ambulatory peritoneal dialysis device (1) comprising a body (6) having suitable areas for hanging a dialysis fluid bag (7a) and a discharge bag (7b), a first weight sensor (4a) where the dialysis fluid bag (7a) is hanged upon which measures the weight of the bag generates a signal proportional thereto, a second weight sensor (4b) where the discharge bag (7b) is hanged upon which measures the weight of the bag generates a signal proportional thereto, and a dialysis fluid valve (2a) and a dialysate valve (2b) provided on said body (6),

- generating an electric signal proportional to the weight of the dialysis fluid bag (7a) by means of the first weight sensor (4a), and closing the dialysis fluid valve (2a) when the signal is equal or less than a predetermined value,

- generating an electric signal proportional to the weight of the discharge bag (7b) by means of a second weight sensor (4b), and closing the dialysate valve (2b) when the signal is equal or greater than a predetermined value,

- generating a filtration data and storing said data in a memory depending on the difference of these signals received from said weight sensors (4a, 4b) by means of a mobile device (3),

This method may additionally comprise the following steps:

- sending the filtration data which are generated by mobile device (3) based upon the signals received from weight sensors (4a, 4b) to a server (101) connected with a database (100) and storing the same in said database (100),

- providing communication of said server (101) with said mobile device (3) and at least one terminal device (102).

## Brief Description of the Figures

Fig. 1 is a representative drawing of a detailed view of the continuous ambulatory peritoneal dialysis device (1) and its valve set (2) according to an embodiment of the present invention.

Fig. 2 is a perspective view of the peritoneal dialysis device (1) as shown in Fig. 1.

Fig. 3 is a perspective view of the heater module (9) which is foreseen to be used in the peritoneal dialysis device (1) in a preferred embodiment of the present invention.

Fig. 4 is a schematic illustration of the system comprising the peritoneal dialysis device (1) of the present invention and an external server (101).

## Detailed Description of the Invention

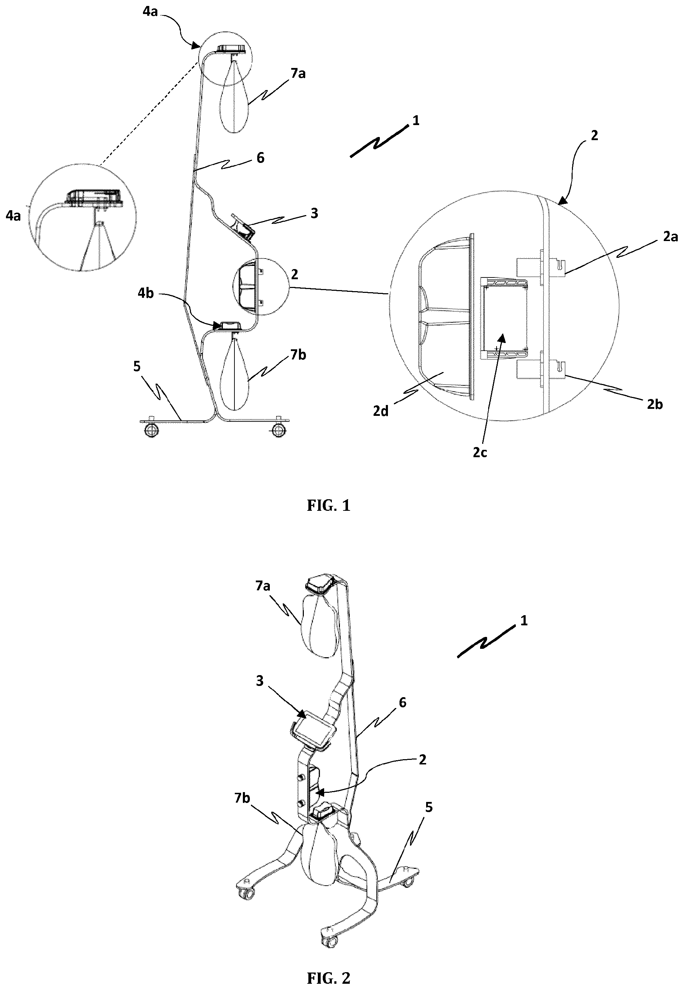

Details of the peritoneal dialysis device (1) according to the present invention are shown in Figures 1 and 2. Said device (1) may have a longitudinal skeletal structure containing a stand (5) and a body (6) section as is known in present devices in the prior art. In such devices, the dialysis fluid bag (7a) is hanged upon a position located above the level of patient so that said fluid can gravimetrically flow into the patient's peritoneal cavity. The dialysate fluid is drained to discharge bag (7b) after completing its function by removing metabolites and the fluid through the peritoneal membrane of patient. This flow takes places by the effect of gravity and the internal pressure of the peritoneal cavity. Continuous ambulatory peritoneal dialysis systems operated by this principle are available in the prior art.

The aim of present invention is to provide said CAPD systems in a form that is more practical, reliable and allowing of medical personnel's control. The peritoneal dialysis device (1) according to the invention may comprise a flow control panel (2). This panel comprises a dialysis fluid valve (2a), a dialysate valve (2b) and at least a processor (2c). Optionally, this device can be concealed with a cover (2d). Although the said components are presented in compact form as a panel, it is also possible for the components to be located separately at suitable locations of the peritoneal dialysis device (1), as would be appreciated by those skilled in the art.

The peritoneal dialysis device (1) according to the present invention comprises a first weight sensor (4a) located at an upper position where the dialysis fluid bag (7a) is hanged upon and a second weight sensor (4b) located at a lower position where the discharge bag (7b) is hanged upon as shown in Figures 1 and 2. Dialysis fluids available in the market are sold as double bags. One of these bags contains a complete dialysis fluid, the other one is provided as empty for discharging of the dialysate fluid. For the dialysis cycle to be initiated in the system according to the present invention, the patient hangs the dialysis fluid bag (7a) to the upper position of patient's level where first weight sensor (4a) is located and the discharge bag (7b) to a below position of patient's level where second weight sensor (4b) is located. Said bags (7a, 7b) are connected to the dialysate valve (2a) and the dialysate valve (2b) by means of tubes (not shown), wherein the patient connects the catheter tip in his/her abdomen to the proper valve during cycles of filling and discharging. Accordingly, in the beginning stage of peritoneal dialysis, the patient firstly connects the catheter tip to the dialysate valve (2a) and fluid flow to the patient's peritoneal cavity begins. After about 6-8 minutes of filling period, the dialysis fluid valve (2a) is closed. After, the necessary filtration for dialysis is performed in the abdominal cavity, drainage of the dirty dialysate fluid is conducted. For this, the patient connects the catheter tip to the dialysate valve (2b) and dialysate fluid starts to flow to the dialysate discharge bag (7b) by activation of the valve.

In an aspect, the present invention relates to a continuous ambulatory peritoneal dialysis device (1) to perform above procedures comprising the components below:

- a body (6) having suitable places for hanging a dialysis fluid bag (7a) and a discharge bag (7b),
- a first weight sensor (4a) measuring weight of the dialysis fluid bag (7a) at the place where this bag is hanged upon and generating a signal proportional thereto,
- a second weight sensor (4b) measuring weight of the discharge bag (7b) at the place where this bag (7b) is hanged upon and generating a signal proportional thereto,
- a dialysis fluid valve (2a) and a dialysate valve (2b) provided on the body (6)
- a processor (2c) generating a control signal to close the dialysis fluid valve (2a) when the signal received from the first weight sensor (4a) is equal or less than a predetermined value and to close the dialysate valve (2b) when the signal received from the second weight sensor (4b) is equal or greater than a predetermined value.

The continuous ambulatory peritoneal dialysis device (1) containing the features presented above, automatically closes the dialysis fluid valve (2a), when the fluid in dialysis fluid bag (7a) is drained or specified amount of fluid flow is maintained, by means of weight data signal generated by the first weight sensor (4a). In this way, excessive dialysis fluid flow to the patient is prevented. In the same way, the dialysis fluid valve (2a) is automatically closed, when the desired amount of dialysate accumulates in the discharge bag (7b), by means of the weight data signal generated by the second weight sensor (4b), whereby excessive fluid drainage from the patient is avoided. Since control of the valves (2a, 2b) is conducted by means of the above described processor (2c), transition between dialysis cycles is not left to the patient's initiative. Thus, a safe and useful peritoneal dialysis process can be carried out.

In the peritoneal dialysis device (1) according to the invention, the mobile device (3) can be any electronic device which has a processor and a memory such as computer, laptop, smart phone or tablet. Said mobile device (3) provides a considerable advantage with respect to the follow up of the treatment by storing the weight data in the memory according to signals received from said weight sensors (4a, 4b). For example, the history of dialysis treatment within a certain period of time can be displayed in graphs, diagrams, or texts during consultancy with a medical doctor who carries out medical follow up of the patient. The treatment compliance and commitment of the patient can be easily understood.

As mentioned above the said mobile device (3) is arranged to receive signals from the weight sensors (4a, 4b) and generates a filtration data from them based on a difference thereof. Herein said filtration data refers to the metabolites, minerals, biological material and fluid material which pass from the abdominal cavity to the dialysate fluid in the patient's peritoneal cavity. Therefore, the difference between the weight ($m_{DSi}$) of the dialysate fluid bag (7a) before start of the dialysis and the weight ($m_{Df}$) of the discharge bag (7b) where dialysate is filled is important. Therefore in an embodiment not according to the invention as claimed, said mobile device (3) comprises a processor adapted to calculate the difference between the initial weight and final weight ($m_{Df}$ - $m_{DSi}$). Here, the said $m_{DSi}$ weight is the weight of filled dialysis fluid bag (7a) before the peritoneal dialysis begins. $m_{Df}$ is the weight of discharge bag (7b) filled with the dirty solution after the peritoneal dialysis cycles are completed. The difference between the two weights mentioned above presents the sum of metabolites, minerals, biological material and fluid filtration that pass from the abdominal cavity to the dialysate fluid in the patient's peritoneal cavity.

According to the invention as claimed, a mobile device (3) is provided, wherein the mobile device (3) is adapted for measuring and processing the data received from said weight sensors (4a, 4b) in a specific manner wherein filtration can be measured more precisely. Accordingly, the mobile device (3), receives the weight of dialysis bag (7a) and the discharge bag (7b) from the weight sensors (4a, 4b) at the beginning and at the end of the dialysis cycles. The measured weights are as follows;

$m_{DSi}$ = weight of the initially filled dialysate fluid bag (7a),

$m_{DSf}$ = weight of the drained dialysis fluid bag (7a),

$m_{Di}$ = weight of the initially empty discharge bag (7b),

$m_{Df}$ = weight of the discharge bag (7b) filled with dialysate.

Accordingly, the above values read from the weight sensors (4a, 4b) are processed by the mobile device (3) by the following calculation method to obtain a filtration weight data $m_F$;

$$(m_{Df} - m_{Di}) - (m_{DSi} - m_{DSf}) = m_F$$

The filtration data calculated in this way is more reliable than the previous method. Because during the peritoneal dialysis, when a valve (2a) is closed and the other valve (2b) is opened, a certain amount of dialysis solution can still be present in the dialysis fluid bag (7a) and this causes a calculation error. The above method completely eliminates this error.

Mobile device (3) according to present invention can store filtration data calculated based on weight signals with time parameters in a memory. As said above, the systematic storage of these data based upon time is extremely important with regard to subsequent evaluation by the medical personnel as to the dialysis performance and patient's commitment to the treatment.

Communication between the mobile device (3) and the weight sensors (4a, 4b) presented in the peritoneal dialysis device (1) according to present invention can be established by any wired or wireless communication means. The same is valid for the processor (2c) which controls the valves (2a, 2b). In case of using wireless communication means, the preferred communication protocol can be Bluetooth, Wi-Fi, Infrared or optical communication means. The signals received from weight sensors (4a, 4b) by means of wired or wireless communication means can be used by the mobile device (3) to calculate filtration data as described above. The same signals as explained above can be used by the processor (2c) in order to control the valves (2a, 2b). However, these operations can also be performed with a single processor as would be appreciated by those skilled in the art, for example with the processor of the mobile device (3).

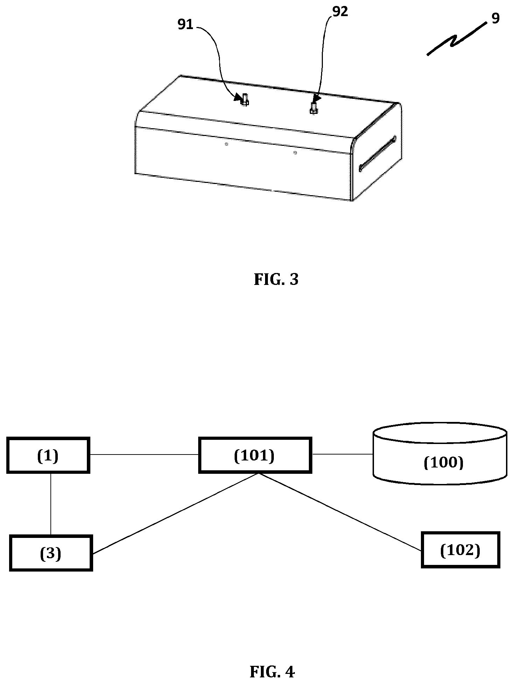

In prior art, it is preferred to heat the dialysis fluid bag (7a) before usage for delivering the dialysis solution to the patient at a convenient temperature. However, the usage of a separate heating device is common for heating these bags. In an advantageous embodiment of the present invention, a heater (9) as shown in Fig. 3 is included in the peritoneal dialysis device (1). Said heater (9), comprises an inlet conduit (91) for the entry of the dialysis solution, and an outlet conduit (92) where the solution is out as heated. In this way, it is possible to heat dialysis solution instantly (in situ) without requiring the patient to preheat the dialysis fluid bag (7a). The heater (9) is preferably located between the dialysis bag (7a) and the dialysis fluid valve (2a) in accordance with the purposes of the present invention. The heater (9) may comprise a heating device operated with electrical resistance, induction or radiation principles. However, the inventor has found that that a heating can be achieved with low electrical consumption in a safe and efficient manner when a halogen lamp is particularly used. Halogen lamps (not shown) provides an effective heating by the heat and radiation they generate when they are located close to an area where the dialysis fluid flows.

In another aspect, present invention relates to a continuous ambulatory peritoneal dialysis system as shown in Fig. 4. Said system comprises a peritoneal dialysis device (1) of the above type having a mobile device (3), a server (101) in communication with said device (1), and at least one terminal device (102) in communication with said server (101). Said server (101) is connected with a database (100). In this system, data sent from the mobile device (3) is transferred to the database (100) and stored therein. In another variation, the peritoneal dialysis device (1) may be in direct communication with said server (101). Consequently, it is possible to visualize the data stored by the patient's mobile device (3) or any terminal device (102), (e.g. doctor's computer). In this context, even if the patient does not have a mobile device (3) or external memory, it is possible for any medical personnel to connect remotely and monitor said data.

In another aspect, the present disclosure further provides a method for collection and processing of continuous ambulatory peritoneal dialysis data comprising the steps of;

- providing a continuous ambulatory peritoneal dialysis device (1) comprising a body (6) having suitable places for hanging a dialysis fluid bag (7a) and discharge bag (7b), a first weight sensor (4a) measuring weight of the dialysis fluid bag (7a) at the place where this bag is hanged upon and generating a signal proportional thereto, a second weight sensor (4b) measuring weight of the discharge bag (7b) at the place where this bag is hanged upon and generating a signal proportional thereto , and a dialysis fluid valve (2a) and a dialysate valve (2b) provided on the body (6).
- generating an electric signal proportional to the weight of the dialysis fluid bag (7a) by means of the first weight sensor (4a), and closing the dialysis fluid valve (2a) when the signal is equal or less than a predetermined value,
- generating an electric signal proportional to the weight of the discharge bag (7b) by means of a second weight sensor (4b), and closing the dialysate valve (2b) when the signal is equal or greater than a predetermined value, and
- generating a filtration data based on a difference of these signals received from said weight sensors (4a, 4b) and storing said data in a memory by means of a mobile device (3),

Other advantageous embodiments and variations of the invention may be contemplated without deviating from the scope of the appended claims.

## Claims

1. A continuous ambulatory peritoneal dialysis device (1) comprising a body (6) having suitable places for hanging a dialysis fluid bag (7a) and a discharge bag (7b), a first weight sensor (4a) configured for measuring weight of the dialysis fluid bag (7a) in the place where this bag is hanged upon and generating a signal proportional thereto, a second weight sensor (4b) configured for measuring weight of the discharge bag (7b) in the place where this bag is hanged upon and generating a signal proportional thereto, and a dialysis fluid valve (2a) and a dialysate valve (2b) provided on said body (6) **characterized in that;**

   the device comprises a processor (2c) configured for generating a control signal to close the dialysis fluid valve (2a) when the signal received from the first weight sensor (4a) is equal to or less than a predetermined value and to close the dialysate valve (2b) when the signal received from the second weight sensor (4b) is equal to or greater than a predetermined value, and a mobile device (3) configured for receiving signals from said weight sensors (4a, 4b) and generating a filtration data based on a difference of signals received from the first weight sensor (4a) and on a difference of signals received from the second weight sensor (4b), said filtration data is a filtration weight ($m_F$), wherein;

   $$m_F = (m_{Df} - m_{Di}) - (m_{DSi} - m_{DSf})$$

   and wherein;

   $m_{DSi}$ = weight of the initially filled dialysate fluid bag (7a),
   $m_{DSf}$ = weight of the drained dialysis fluid bag (7a),
   $m_{Di}$ = weight of the initially empty discharge bag (7b),
   $m_{Df}$ = weight of the discharge bag (7b) filled with dialysate.

2. A continuous ambulatory peritoneal dialysis device (1) according to claim 1 wherein said mobile device (3) comprises a memory for storing time dependent filtration data.

3. A continuous ambulatory peritoneal dialysis device (1) according to claim 1 wherein said mobile device (3) is a computer, laptop, smart phone or tablet.

4. A continuous ambulatory peritoneal dialysis device (1) according to claim 1 wherein communication between said mobile device (3) and weight sensors (4a, 4b) is provided with a wireless communication means comprising Bluetooth, Wi-Fi, Infrared or optical communication protocols.

5. A continuous ambulatory peritoneal dialysis device (1) according to claim 1 wherein communication between said processor (2c) and weight sensors (4a, 4b) is provided with a wireless communication means comprising Bluetooth, Wi-Fi, Infrared or optical communication protocols.

6. A continuous ambulatory peritoneal dialysis device (1) according to claim 1 wherein said dialysis device (1) further comprises a heater (9).

7. A continuous ambulatory peritoneal dialysis device (1) according to claim 6 wherein said heater (9) comprises a heating device operated with electrical resistance, induction or radiation principles.

8. A continuous ambulatory peritoneal dialysis device (1) according to claim 7 wherein said heater (9) comprises a halogen lamp.

9. A continuous ambulatory peritoneal dialysis system comprising a continuous ambulatory peritoneal dialysis device (1) according to claim 1, a server (3) in communication with the device (1), a database (100) connected to said server (3) and at least one terminal device (102) in communication with said server (3), the server (3) preferably being in communication with the mobile device (3) of the continuous ambulatory peritoneal dialysis device (1).

10. A continuous ambulatory peritoneal dialysis system according to claim 9 wherein said database (100) comprises time dependent filtration data.

11. A continuous ambulatory peritoneal dialysis system according to claim 9 wherein the continuous ambulatory peritoneal dialysis device (1) and/or terminal device (102) comprises an internet communication protocol for communication with the server (3), preferably the mobile device (3) of the continuous ambulatory peritoneal dialysis device (1) comprising an internet communication protocol for communication with the server (3).

**Patentansprüche**

1. Kontinuierliche ambulante Peritonealdialysevorrichtung (1), umfassend einen Körper (6) mit geeigneten Stellen zum Aufhängen eines Dialyseflüssigkeitsbeutels (7a) und eines Auslassbeutels (7b), einen ersten Gewichtssensor (4a), der zum Messen des Gewichts des Dialyseflüssigkeitsbeutels (7a) an der Stelle, an der dieser Beutel aufgehängt ist, und zum Erzeugen eines dazu proportionalen Signals konfiguriert ist, einen zweiten Gewichtssensor (4b), der so konfiguriert ist, dass er das Gewicht des Auslassbeutels (7b) an der Stelle, an der dieser Beutel aufgehängt ist, misst und ein dazu proportionales Signal erzeugt, und ein Dialyseflüssigkeitsventil (2a) und ein Dialysatventil (2b), die an dem Körper (6) vorgesehen sind, **dadurch gekennzeichnet, dass;**

die Vorrichtung einen Prozessor (2c) umfasst, der zum Erzeugen eines Steuersignals konfiguriert ist, um das Dialyseflüssigkeitsventil (2a) zu schließen, wenn das von dem ersten Gewichtssensor (4a) empfangene Signal gleich oder kleiner als ein vorbestimmter Wert ist, und um das Dialysatventil (2b) zu schließen, wenn das von dem zweiten Gewichtssensor (4b) empfangene Signal gleich oder größer als ein vorbestimmter Wert ist, und
eine mobile Vorrichtung (3), die zum Empfangen von Signalen von den Gewichtssensoren (4a, 4b) und zum Erzeugen von Filtrationsdaten auf der Grundlage einer Differenz von Signalen, die von dem ersten Gewichtssensor (4a) empfangen werden, und auf der Grundlage einer Differenz von Signalen, die von dem zweiten Gewichtssensor (4b) empfangen werden, konfiguriert ist,
wobei die Filtrationsdaten ein Filtrationsgewicht ($m_F$) sind, wobei;

$$m_F = (m_{Df} - m_{Di}) - (m_{Dsi} - m_{DSf})$$

und wobei:

$m_{Dsi}$ = Gewicht des anfänglich gefüllten Dialysatflüssigkeitsbeutels (7a),
$m_{DSf}$ = Gewicht des entleerten Dialyseflüssigkeitsbeutels (7a),
$m_{Di}$ = Gewicht des anfänglich leeren Auslassbeutels (7b),
$m_{Df}$ = Gewicht des mit Dialysat gefüllten Auslassbeutels (7b).

2. Kontinuierliche ambulante Peritonealdialysevorrichtung (1) nach Anspruch 1, wobei die mobile Vorrichtung (3) einen Speicher zum Speichern zeitabhängiger Filtrationsdaten umfasst.

3. Kontinuierliche ambulante Peritonealdialysevorrichtung (1) nach Anspruch 1, wobei die mobile Vorrichtung (3) ein Computer, Laptop, Smartphone oder Tablet ist.

4. Kontinuierliche ambulante Peritonealdialysevorrichtung (1) nach Anspruch 1, wobei die Kommunikation zwischen der mobilen Vorrichtung (3) und den Gewichtssensoren (4a, 4b) mit einem drahtlosen Kommunikationsmittel erfolgt, das Bluetooth, Wi-Fi, Infrarot oder optische Kommunikationsprotokolle umfasst.

5. Kontinuierliche ambulante Peritonealdialysevorrichtung (1) nach Anspruch 1, wobei die Kommunikation zwischen dem Prozessor (2c) und den Gewichtssensoren (4a, 4b) mit einem drahtlosen Kommunikationsmittel bereitgestellt wird, das Bluetooth-, Wi-Fi-, Infrarot- oder optische Kommunikationsprotokolle umfasst.

6. Kontinuierliche ambulante Peritonealdialysevorrichtung (1) nach Anspruch 1, wobei die Dialysevorrichtung (1) ferner eine Heizung (9) umfasst.

7. Kontinuierliche ambulante Peritonealdialysevorrichtung (1) nach Anspruch 6, wobei die Heizvorrichtung (9) eine mit elektrischem Widerstand, Induktion oder Strahlung betriebene Heizvorrichtung umfasst.

8. Vorrichtung zur kontinuierlichen ambulanten Peritonealdialyse (1) nach Anspruch 7, wobei die Heizvorrichtung (9) eine Halogenlampe umfasst.

9. Kontinuierliches ambulantes Peritonealdialysesystem, umfassend eine kontinuierliche ambulante Peritonealdialysevorrichtung (1) nach Anspruch 1, einen Server (3), der mit der Vorrichtung (1) in Verbindung steht, eine Datenbank (100), die mit dem Server (3) verbunden ist, und mindestens ein Endgerät (102), das mit dem Server (3) in Verbindung steht, wobei der Server (3) vorzugsweise mit der mobilen Vorrichtung (3) der kontinuierlichen ambulanten Peritonealdialysevorrichtung (1) in Verbindung steht.

10. Kontinuierliches ambulantes Peritonealdialysesystem nach Anspruch 9, wobei die Datenbank (100) zeitabhängige Filtrationsdaten umfasst.

11. Kontinuierliches ambulantes Peritonealdialysesystem nach Anspruch 9, wobei die kontinuierliche ambulante Peritonealdialysevorrichtung (1) und/oder das Endgerät (102) ein Internet-Kommunikationsprotokoll zur Kommunikation mit dem Server (3) umfasst, wobei vorzugsweise die mobile Vorrichtung (3) der kontinuierlichen ambulanten Peritonealdialysevorrichtung (1) ein Internet-Kommunikationsprotokoll zur Kommunikation mit dem Server (3) umfasst.

## Revendications

1. Dispositif de dialyse péritonéale continue ambulatoire (1) comprenant un corps (6) ayant des emplacements appropriés pour suspendre une poche de fluide de dialyse (7a) et une poche de décharge (7b), un premier capteur de poids (4a) configuré pour mesurer le poids de la poche de fluide de dialyse (7a) à l'emplacement où cette poche est suspendue à ce dernier et générer un signal proportionnel à cette dernière, un deuxième capteur de poids (4b) configuré pour mesurer le poids de la poche de décharge (7b) à l'emplacement où cette poche est suspendue à ce dernier et générer un signal proportionnel à cette dernière, et une valve de fluide de dialyse (2a) et une valve de dialysat (2b) prévues sur ledit corps (6), **caractérisé en ce que** :

   le dispositif comprend un processeur (2c) configuré pour générer un signal de commande afin de fermer la valve de fluide de dialyse (2a) lorsque le signal reçu du premier capteur de poids (4a) est égal ou inférieur à une valeur prédéterminée et pour fermer la valve de dialysat (2b) lorsque le signal reçu du deuxième capteur de poids (4b) est égal ou supérieur à une valeur prédéterminée, et
   un dispositif mobile (3) configuré pour recevoir des signaux desdits capteurs de poids (4a,4b) et générer des données de filtration sur la base d'une différence des signaux reçus du premier capteur de poids (4a) et d'une différence des signaux reçus du deuxième capteur de poids (4b),
   lesdites données de filtrations sont un poids de filtration ($m_F$), dans lequel :

   $$m_F = (m_{Df} - m_{Di}) - (m_{DSi} - m_{DSf})$$

   et dans lequel :

   $m_{DSi}$ = poids de la poche de fluide de dialysat initialement remplie (7a),
   $m_{DSf}$ = poids de la poche de fluide de dialyse évacuée (7a),
   $m_{Di}$ = poids de la poche de décharge initialement vide (7b),
   $m_{Df}$ = poids de la poche de décharge (7b) remplie avec le dialysat.

2. Dispositif de dialyse péritonéale continue ambulatoire (1) selon la revendication 1, dans lequel ledit dispositif mobile (3) comprend une mémoire pour stocker les données de filtration dépendantes du temps.

3. Dispositif de dialyse péritonéale continue ambulatoire (1) selon la revendication 1, dans lequel ledit dispositif mobile (3) est un ordinateur, un ordinateur portable, un smartphone ou une tablette.

4. Dispositif de dialyse péritonéale continue ambulatoire (1) selon la revendication 1, dans lequel la communication entre ledit dispositif mobile (3) et les capteurs de poids (4a, 4b) est fournie avec un moyen de communication sans fil comprenant le Bluetooth, le Wi-Fi, les protocoles de communication infrarouge ou optique.

5. Dispositif de dialyse péritonéale continue ambulatoire (1) selon la revendication 1, dans lequel la communication entre ledit processeur (2c) et les capteurs de poids (4a, 4b) est fournie avec un moyen de communication sans fil comprenant le Bluetooth, le Wi-Fi, les protocoles de communication infrarouge ou optique.

6. Dispositif de dialyse péritonéale continue ambulatoire (1) selon la revendication 1, dans lequel ledit dispositif de dialyse (1) comprend en outre un dispositif de chauffage (9).

7. Dispositif de dialyse péritonéale continue ambulatoire (1) selon la revendication 6, dans lequel ledit dispositif de chauffage (9) comprend un dispositif de chauffage actionné avec des principes de résistance électrique, d'induction ou de rayonnement.

8. Dispositif de dialyse péritonéale continue ambulatoire (1) selon la revendication 7, dans lequel ledit dispositif de chauffage (9) comprend une lampe halogène.

9. Système de dialyse péritonéale continue ambulatoire comprenant un dispositif de dialyse péritonéale continue ambulatoire (1) selon la revendication 1, un serveur (3) en communication avec le dispositif (1), une base de données (100) raccordée audit serveur (3) et au moins un dispositif terminal (102) en communication avec ledit serveur (3), le serveur (3) étant de préférence en communication avec le dispositif mobile (3) du dispositif de dialyse péritonéale continue ambulatoire (1).

10. Système de dialyse péritonéale continue ambulatoire selon la revendication 9, dans lequel ladite base de données (100) comprend des données de filtration dépendantes du temps.

11. Système de dialyse péritonéale continue ambulatoire selon la revendication 9, dans lequel le dispositif de dialyse péritonéale continue ambulatoire (1) et/ou le dispositif terminal (102) comprend (comprennent) un protocole de communication internet pour la communication avec le serveur (3), de préférence le dispositif mobile (3) du dispositif de dialyse péritonéale continue ambulatoire (1) comprenant un protocole de communication internet pour la communication avec le serveur (3).

**FIG. 1**

**FIG. 2**

91     92                    9

FIG. 3

| (1) |——| (101) |——| (100) |

| (3) |

| (102) |

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160216150 A